# EUROPEAN PATENT SPECIFICATION

(11) **EP 2 129 312 B1**
(45) Date of publication and mention of the grant of the patent: **14.05.2014**
(21) Application number: 08732157.6
(22) Date of filing: 13.03.2008
(51) Int. Cl.: A61B 18/14, A61B 18/16, A61B 18/12, A61B 17/00, A61B 18/00, A61B 19/00

(54) **APPARATUS FOR MODERATING RETURN ELECTRODE TEMPERATURE**
GERÄT ZUR KONTROLLE DER TEMPERATUR EINER NEUTRALELEKTRODE
APPAREIL POUR MODÉRER UNE TEMPÉRATURE D'ÉLECTRODE DE RETOUR

(30) Priority: 13.03.2007 US 717920; 21.01.2008 US 17272; 21.01.2008 US 17278; 21.01.2008 US 17282
(43) Date of publication of application: 09.12.2009
(73) Proprietor: Halt Medical, Inc., Brentwood, CA 94513 (US); Epstein, Gordon, Freemont, CA 94539 (US)
(72) Inventor: EPSTEIN, Gordon, Freemont, CA 94539 (US)
(74) Representative: Nederlandsch Octrooibureau
(86) International application number: PCT/US2008/056907
(87) International publication number: WO 2008/112931

(56) References cited:
- WO-A2-2005/110263
- US-A- 5 122 137
- US-A- 5 846 238
- US-A1- 2002 111 615
- US-A1- 2005 101 947
- US-A1- 2006 200 120

## Description

### TECHNICAL FIELD

The invention relates to apparatus for preventing return electrodes in a radio frequency ablation system (such as a uterine fibroid ablation system) from overheating and causing injury or discomfort to the patient.

### BACKGROUND OF THE INVENTION

There are a number of applications in which radio frequency ablation is a preferred procedure. Such applications include the treatment of various growths and lesions, such as prostate cancer, liver cancer, and uterine fibroids.

Such techniques generally involve the application of power between a return electrode and an ablation device, such as a pointed trocar tip with a plurality of extendable ablation stylets. A potential complication during the deployment and use of such devices is overheating in the area surrounding the return electrodes, with attendant discomfort and, potentially, injury.

Relevant prior art is provided by WO 2005/110263.

### SUMMARY OF THE INVENTION

In accordance with the invention, an apparatus is provided to reduce the likelihood of return electrode overheating.

In accordance with the invention, which is defined by claim 1, an ablation system comprises a source of electrical ablation energy having first, second and third power outputs. A first conductor is coupled to the first power output on the source of electrical energy. A second conductor is coupled to the second power output on the source of electrical energy, the source of electrical energy creates a first output ablation voltage between the first and second power outputs. The first output ablation voltage varies between a first higher average value during a first period of time and a first lower average value for a second period of time. The first lower average value is greater than or equal to zero. A third conductor is coupled to a third power output on the source of electrical energy. The source of electrical energy creates a second output ablation voltage between the first and third power outputs. The second output ablation voltage varies between a second higher average value during a third period of time and a lower average value for a fourth period of time. The lower average value is greater than or equal to zero. An ablation probe is coupled to the first conductor.

An electrode provides a return path for an ablation device. The electrode comprises a first conductive ablation member which defines a first contact surface. The first contact surface defines a first active peripheral edge on a first active side of the first conductive member. A first coupling member is coupled to the second conductor and electrically connected to a first power coupling edge of the first conductive member. The second edge is an edge of the first conductive member other than the first active peripheral edge. The first coupling member is made of an electrically conductive material. A second conductive ablation member and defines a second contact surface, the second contact surface defines a second active peripheral edge on a second active side of the second conductive member. A second coupling member coupled to the third conductor is electrically connected to a second power coupling edge of the second conductive member. The second power coupling edge is an edge of the second conductive member other than the second active peripheral edge. The second coupling member is made of an electrically conductive material. The first active peripheral edge is positioned between the first power coupling edge and the second power coupling edge.

In accordance with the inventive system, the first period of time may overlap a substantial portion of the fourth period of time, and the second period of time may overlap a substantial portion of the third period of time.

The power coupling edges may be opposite the active peripheral edges.

In a preferred embodiment, the peripheral edges are substantially straight and have first and second ends, and a curved edge is contiguous to each of the first and second ends.

In accordance with an exemplary method of ablating a biological body in a mammal, ablation energy is applied between an ablation stylet and, intermittently, first and second skin electrodes.

Generally, the same may also be achieved by, alternatively or additionally, monitoring electrode temperature and varying either the electrodes which are being driven with radio frequency ablation energy, and/or varying the amount of radio frequency ablation energy being coupled to the electrodes.

In accordance with the invention, it is recognized that RF current flow is concentrated at the leading edge of the pad (the cephalic edge of the pad in the case of uterine fibroid ablation). Hence, temperature rises in the tissue proximate the leading edge. There may be a plurality of pads with single or multiple electrodes on a single substrate. Each electrode is thus associated with a zone of skin positioned underneath it switching between electrode which overlie different zones may be referred to as zone switching. Zone switching is done to essentially create a new leading edge as the old one warms up. Pad zone switching is intended to distribute the heating that occurs on the skin under a return electrode by changing the zone that is being used. The majority of the heating occurs along the leading edge of a return electrode because the path from that edge to the ablation site is shortest and thus has the lowest impedance. By, in effect, moving that leading edge around, local heating is reduced, minimizing the probability of a burn.

In a typical arrangement return electrode pads having one or more electrodes are located on both of the patient's legs. If each leg has a single return electrode pad divided into two or three electrically isolated electrodes and corresponding zones, the zones may be oriented across the leg, like stripes. If at one point in time the first (e.g. cephalic) zone is in use as the return electrode, heating occurs primarily on the leading edge of that zone. A short time later (e.g. ten seconds) the system can switch to another electrode associate with a different zone. That moves the localized heating to the leading edge of the new zone and gives the body time to cool the previously-used zone. Blood flow and conduction will tend to bring the skin under the "old" leading edge back to normal body temperature. In effect, we are moving the return electrode from one place to another and are avoiding prolonged heating of a single patch of skin.

Generally, effective cooling is achieved because power is maintained at levels where the time it takes for a zone to increase in temperature by a certain amount is reliably a little longer than the time it takes for the body to cool that zone back down after the zone is changed in the case of a two electrode arrangement. If there are more than two electrodes, more elaborate methodologies may be employed, as detailed below.

One possible algorithm involves, for each leg, starting to apply RF using (as the return electrode) the coldest of the N zones on that leg. After a certain time (e.g. five seconds), the system starts looking at the temperature along the leading edge of the zone(s) in use. If the temperature reaches the "switching" level (e.g. 40°C) the system then switches to the coldest zone. In any case, if a maximum time limit (e.g. thirty seconds) is reached and the temperature has not yet reached the switching level, then the system switches to the coldest zone. Note that if the zone being currently excited is still the coldest of the N zones there is no change in zone, as the object is to use the coldest zone at all times.

As an alternative to using the coolest zone or other selection strategy, the system may be designed to periodically change zones, for example every thirty seconds to always implement a switch in active pad to act as a fail safe for a pad that happens to lift right where a sensing thermocouple is placed. The thermocouples are preferably placed at the cephalic midline of the zone to make it less likely that it will peel up there, but if it did and there is not enough of a change in impedance to trigger a contact quality alarm threshold, by moving the active zone every set number of seconds, a risk of injury is mitigated.

The exemplary method for ablating a tissue mass associated with a human or animal patient being treated comprises positioning an ablating electrode in a tissue mass to be ablated. A plurality of return electrodes are positioned on the patient. Electrical energy is applied between the return electrodes and the ablating electrode. The temperature of the return electrodes is measured to generate a temperature measurement signal.

The electrical energy applied between the return electrodes and the ablating electrode may be varied in response to the temperature measurement signal.

The electrical energy may be radio frequency energy.

The temperature may be measured on a portion of the return electrode which may be more proximate to the tissue mass than other portions of the return electrode. The temperature may be measured on an edge of the return electrode.

The varying of electrical energy may comprise shutting off one or more electrodes. The varying of electrical energy may comprise apportioning electrical energy between the electrodes. The apportionment may be made by sending more electrical energy to electrodes which are less likely to become overheated. The electrical energy may be apportioned by varying the duty cycle of electrical energy sent to each of the return electrodes.

The apportionment may be made by sending more electrical energy to electrodes which are more likely to cool rapidly, proportionately on the basis of cooling rate. The apportionment may be made by sending more electrical energy to electrodes which are less likely to become overheated and by sending more electrical energy to electrodes which are more likely to cool rapidly, proportionately on the basis of cooling rate.

The impedance path of return electrodes may be measured to determine the existence of a poor electrical connection.

In accordance with the invention, a device is provided for ablating tissue masses associated with a human or animal patient, the invented device comprises an electrical source for generating an ablation current. A coupling circuit is coupled to the electrical source. An ablation electrode is coupled to the coupling circuit. A plurality of return electrodes are coupled to the coupling circuit. A plurality of temperature measurement transducers are each associated with a respective return electrode. The temperature transducers each provide a temperature measurement signal. The computing device is coupled to the temperature measurement signals for producing signals to control the coupling of the return electrodes to the coupling circuit.

An initial set of operating parameters may be deployed. These operating parameters may be varied in response to heating and/or cooling of the electrodes.

The inventive apparatus may include software for storing information for operation type, or surgeon identity, or another factor to generate initial parameters for the system.

In an exemple, the amount of energy sent to a return electrode is varied individually as a function of its temperature history.

The temperature measurement transducers may be secured to their respective return electrodes.

The amount of energy sent to a return electrode may be varied individually.

The apparatus may provide a display of electrode temperature in a first color, such as green or blue, when the return electrode is cool, and a second color, such as amber, when the return electrode is becoming significantly warmed, and in a third color, such as red, when the return electrode has exceeded acceptable threshold temperature.

The apparatus may provide for the amount of ablation current being sent to one of the return electrodes to be varied as a function of temperature in accordance with a first algorithm, and the amount of ablation current being sent to another of the return electrodes to be varied as a function of temperature in accordance with a second algorithm, with the second algorithm being different from the first algorithm.

The apparatus may include software for storing information for operation type, or surgeon identity, or another factor to generate initial parameters for the system.

### BRIEF DESCRIPTION THE DRAWINGS

The operation of the apparatus will become apparent from the following description taken in conjunction with the drawings, in which:
FIG.1 is a flow chart generally illustrating one exemplary implementation of operating the apparatus of the present invention;
FIG. 2 is a block diagram illustrating an alternative exemplary method of operating the apparatus of the present invention;
FIG. 3 illustrates another exemplary method of operation;
FIG. 4 is a flow chart of yet another exemplary method of operation;
FIG. 5 illustrates still yet another alternative in which both power and the number of electrodes is varied;
FIG. 6 illustrates an algorithm for varying and allocating power between electrodes;
FIG. 7 illustrates an apparatus of the present invention;
FIG. 8 illustrates a top plan view of an electrode according to the present invention;
FIG. 9 is a side view along lines 9-9 of Figure 8;
FIG. 10 illustrates the placement of electrodes;
Figures 11-14 illustrate alternative electrodes;
Figure 15 illustrates an alternative examplary method of operation;
Figure 16 illustrates electrode placement on the thighs of a subject;
Figure 17 is a diagram illustrating the heating and cooling of the body adjacent a return electrode pad;
Figure 18 is a schematic representation of a known return path electrode pad;
Figure 19 is a schematic representation of an alternative return path electrode pad;
Figure 20 is a schematic representation of another alternative return path electrode pad; and
Figure 21 is a schematic representation of still another alternative return path electrode pad.

### DETAILED DESCRIPTION OF THE PREFERRED EMBODIMENTS

Typically, ablation is performed using a device comprising an elongated handle which may be gripped by the surgeon. Extending from the handle is a thin elongated, often pointed sometimes stiff but somewhat flexible catheter which may be used to puncture the skin and enter the region where the particular growth or lesion to be removed is located.

The above device is of particular value with respect to the ablation of uterine fibroids. Alternatively, a device with a rounded tip may be used to implement ablation in parts of the body that allow entrance without piercing, for example the urethra. Other areas of application include liver lesions, prostate cancer, and so forth. The present invention applies to all of the above and similar devices.

In such devices a plurality of stylets extends from the pointed tip and may be deployed into a growth to be removed. For example, the tip, under the guidance of ultrasound imaging may be advanced into a liver lesion. Stylets extending from the tip are then deployed through holes in the tip and positioned in various parts of the lesion to be ablated. Typically, the stylets are electrically conductive wires which are housed within the tip and under the action of a lever, slider or other device in the handle of the ablation instrument are advanced through those holes and into the growth to be ablated.

The application of radio frequency energy to the stylets is controlled by another control located on the handle of the ablation device, such as a radio frequency energy actuation button. For example, this may be a push button control. The application of RF energy may be continuous, or pulsating with a fixed or variable duty cycle.

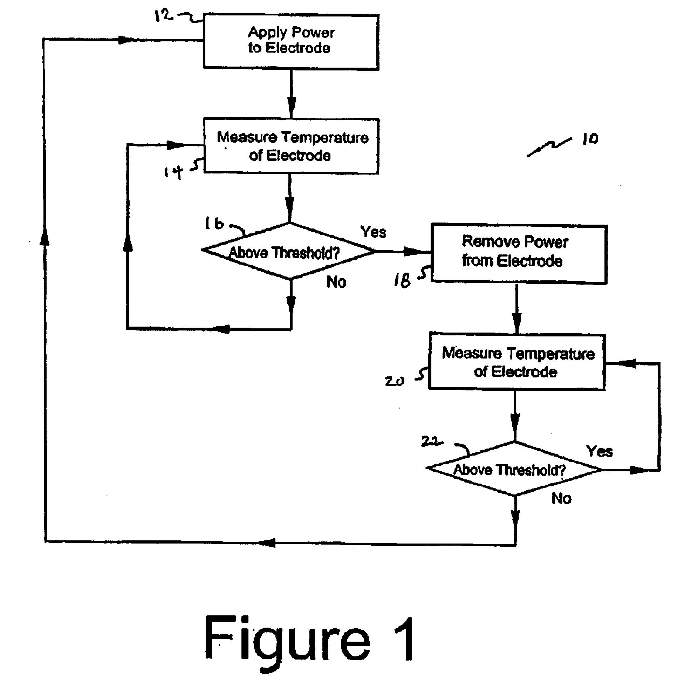

Referring to Figure 1, a method 10 for controlling the temperature of electrodes and preventing overheating is illustrated. In accordance with method 10, power is applied to a return electrode at step 12. The temperature of the electrode is measured at step 14. If the temperature of the electrode exceeds an acceptable threshold value as determined at step 16, the system removes power from the electrode at step 18.

If the temperature of the electrode is not in excess of the maximum acceptable electrode temperature, the system proceeds back to step 14 to measure electrode temperature.

Periodically, the electrode temperature is measured by the system automatically at step 20. Measurement of the temperature of electrode may be done by briefly removing ablation power from the return electrode. During the period in which ablation power has been removed from the return electrode, the system reads the temperature measured by a temperature transducer, whose structure is more fully described below. The removal of power allows the relatively weak signals produced by the temperature transducer to be read, without the interference of the relatively high power RF ablation signal applied to the return electrode. If the measured temperature is still above the threshold, at step 22 the system proceeds back to step 20 to repeat the measurement. If, on the other hand, the temperature is below the threshold, at step 22 the system returns power to the electrode at step 12.

It is contemplated that the above control scheme may be applied in sequence and repeatedly to each of the return electrodes, thus ensuring that none of them overheat.

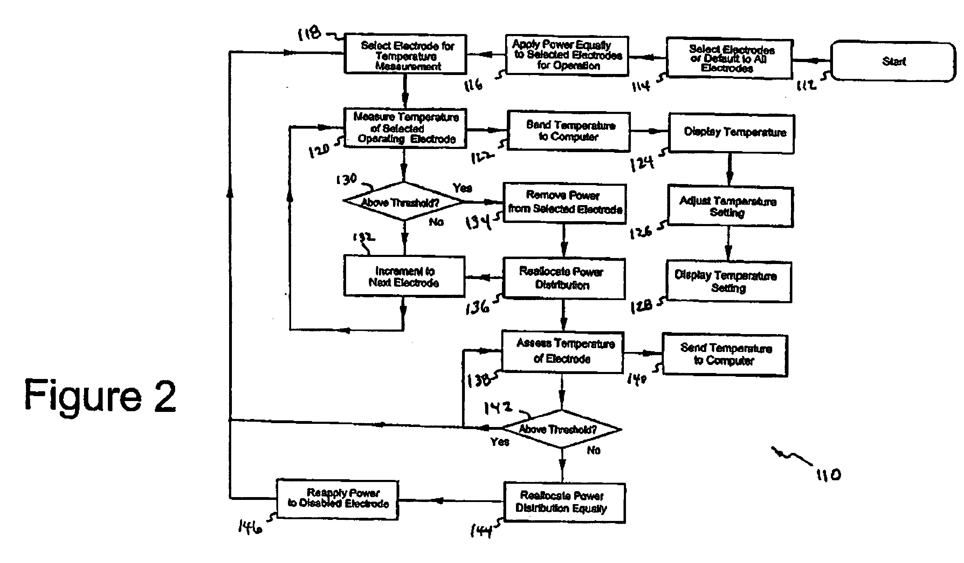

An alternative method 110 is illustrated in Figure 2. The system starts at step 112 to apply power. At step 114, the surgeon is given the option of using all electrodes or selecting only certain electrodes. At step 116, equal power is applied to all electrodes. At step 118 one electrode is selected for temperature measurement. Such measurement is made at step 120 and sent to the computer at step 122. The measured temperature is displayed at step 124. The surgeon is also given the opportunity, which is implemented at step 126, to change or select a maximum operating temperature for the return electrodes. The selection is illustrated at step 128. Different threshold temperatures, optionally, may be selected for different electrodes.

If the temperature measured at step 120 is not above the selected threshold, at step 130, the system proceeds to step 132, where it increments to the next electrode and the temperature of that electrode is measured at step 120. It is contemplated that temperature measurements may be made when all RF power is removed from all of the return electrodes and the system is in a measurement mode. While, in principle, it is possible to take a temperature measurement most easily with only one electrode, namely that electrode whose temperature is to be measured, disconnected from the RF ablation power source, additional reliability is provided by disconnecting all ablation power during measurement. Because measurements may be taken in a very short period of time, for example on the order of milliseconds, there is no noticeable effect on the ablation procedure on account of the disablement of ablation power during measurement.

If the temperature measured at step 120 is above the selected threshold, at step 130 the system proceeds to step 134 where control signals to remove power from the electrode are generated by the system. The power distribution between the electrodes is thus reorganized at step 136. The system then proceeds periodically to step 138 where the temperature of the disabled electrode is periodically checked. As noted above, the temperature of all return electrodes may be periodically checked during a single measurement period. This temperature is sent to the computer at step 140, for example, for display, and to provide the surgeon with an opportunity to override the same. If the temperature is found to be above the threshold at step 142, the system proceeds back to step 138. The system also returns to step 118 to repeat the cycle.

On the other hand, if the temperature is not above the threshold, the system proceeds to step 144 where power between the electrodes is distributed equally and, at step 146 power is applied to the previously disabled electrode, allowing the system to return to step 118 to repeat the cycle. Optionally, power may be distributed unequally, as described below.

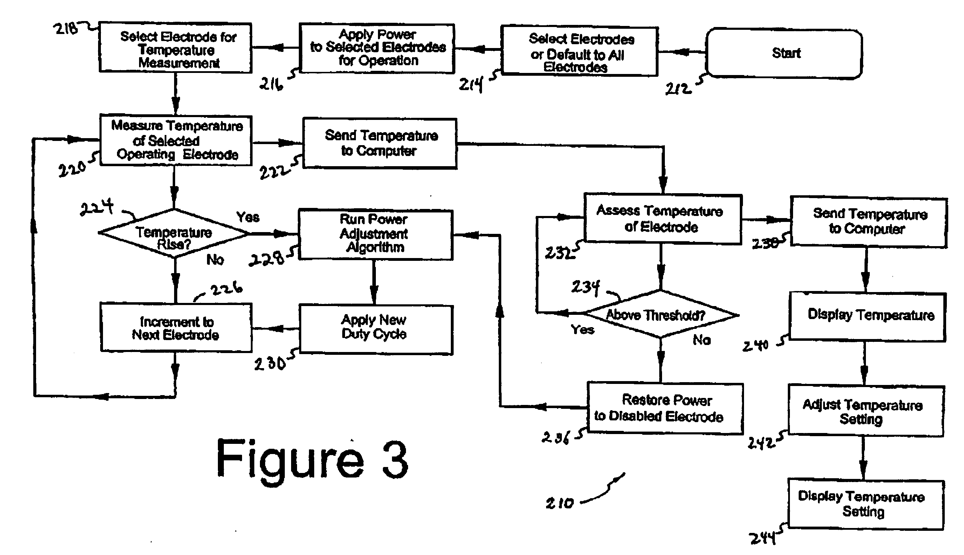

Turning to Figure 3, another alternative exemplary method 210 is illustrated. Method 210 begins at step 212 and provides the opportunity of selecting electrodes at step 214. Power is then applied to the selected electrodes at step 216.

An electrode is then selected for temperature measurement at step 218. Such measurement is made at step 220 and the measured temperature is sent to a computer which controls operation of the system at step 222. If there is no change in the temperature of any electrode, at step 224 the system increments to the next electrode at step 226 and returns to step 220 to repeat the process for all electrodes.

If it is determined at step 224 that there has been a rise in temperature, the system proceeds to step 228 to adjust the power sent to the electrodes in accordance with a power adjustment algorithm. Such adjustment may simply mean applying power only to the two coolest of the four available operating electrodes. Alternatively, the algorithm may simply provide for the hottest electrode to be disabled, thus allowing blood flow to cool the area before discomfort or injury occurs. Alternatively, power may only be applied to the half or two-thirds coolest electrodes.

After the adjustment has been made, the same may be implemented at step 230, after which the system proceeds to the next electrode at step 226. Alternatively, it is possible for power to the electrodes to be varied, by, for example, driving the electrodes with an intermittent RF ablation signal, reducing the duty cycle of the intermittent RF signal to reduce power. Another way to reduce power is to vary the amplitude of a continuous RF signal.

In accordance with the method illustrated in Figure 3, the temperature of the electrodes may also be optionally assessed at step 232. If it is determined that the temperature of an electrode which has been disabled has dropped below the threshold at step 234, power is restored to the disabled electrode at step 236 and the system is returned to step 228 to adjust the allocation of power between electrodes.

If the temperature is still above the threshold, the system returns to step 232 to continue its periodic checks in accordance with a determined schedule. In accordance with the invention, it is contemplated that such a schedule may be as simple as checking temperature every five or 10 seconds. In such a method, all RF energy would be removed from all return electrodes for a period of, for example, 100 ms, during which the system would sequentially, or simultaneously measure the temperature of all electrodes, and reallocate the distribution as described herein. Alternatively, this period may be varied to become more frequent, if prior readings indicated relatively large temperature rises.

In accordance of the invention, the temperature assessed at step 232 is sent to the computer controlling the system at step 238 allowing display of temperature at step 240. Likewise, if desired, the physician may vary the threshold at step 242 with the system displaying the threshold value at step 244.

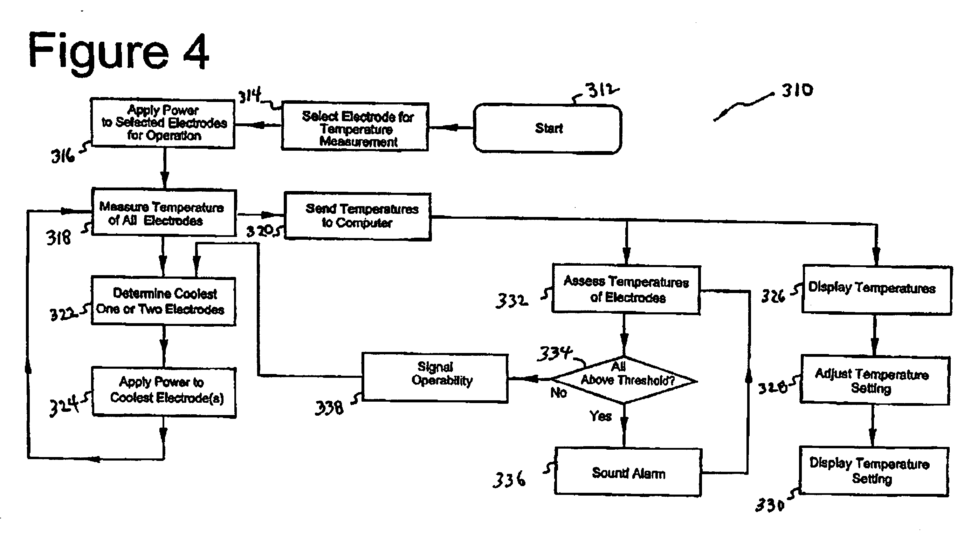

Referring to Figure 4, yet another alternative exemplary method 310 is illustrated. The method begins at step 312 upon the activation of the system. The surgeon then selects electrodes for measurement at step 314 and applies power to the selected electrodes at step 316.

At step 318 the temperatures of all electrodes are measured. At step 320 this information is sent to the computer controlling the system. At step 322 the system determines which electrodes are coolest and drives the coolest or the two coolest electrodes at step 324, provided that they are below the maximum tolerable return electrode temperature. The system then returns to step 318 to check electrode temperatures.

The system displays electrode temperatures at step 326. The system also provides for the adjustment of the maximum tolerable electrode temperature at step 328. This temperature setting is displayed at step 330.

Periodically the system checks electrode temperature at step 332. If all the electrodes have exceeded the maximum tolerable electrode temperature threshold, at step 334, the system determines this and causes the actuation at step 336 of an alarm. After the alarm is actuated the system continues to assess temperature at step 332 and proceed through the temperature checking loop, as described above, until at least one electrode has dropped below the temperature threshold. The fact that the electrode is cool enough to receive energy is determined at step 334 which causes the system to signal that the system is again ready to deliver RF ablation energy. The signal is produced at step 338, after which the system proceeds to step 322 where the coolest one or two electrodes are found and identified as suitable for conducting current, which occurs in step 324.

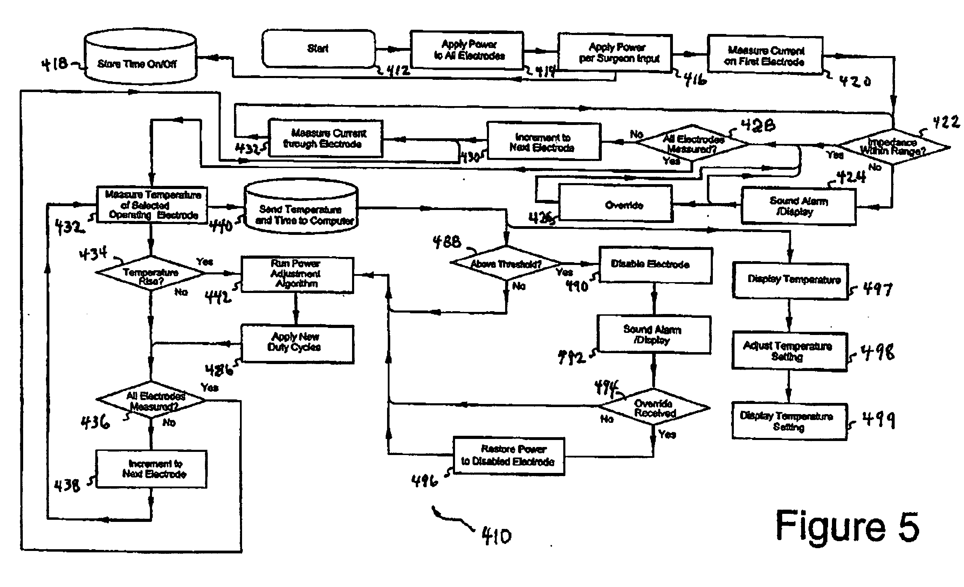

Figure 5 illustrates an exemplary method 410, which may be implemented on a personal or other general-purpose computer, as will be described below. This system both selects electrodes and varies the effective power with which they are driven. The inventive method and apparatus begin operation at step 412 which triggers the application of power to all electrode systems at step 414.

Each time the surgeon activates the application of radio frequency energy, for example by pushing a button on the handle of the ablation apparatus, RF power is applied to the stylets, causing them to apply energy to the tissue surrounding the stylets, with the objective of heating that tissue and killing the cells in the tissue mass to be destroyed, thus allowing the body to remove the dead cells that comprise the growth. Actual generation of RF ablation energy to cause such cell necrosis is triggered by the surgeon at step 416.

The application of radio frequency energy to a growth to be ablated is achieved by the connection of the ablation stylet and a return electrode to the RF energy source. Typically, the return electrode is a planar conductive member which is adhered to the skin of a patient. An electrical path thus exists from the ablation stylet or stylets, through the body to the return path electrode. The return path electrode is typically placed at a position remote from that being treated, for example, on the thighs. In such an arrangement, the much greater area of the return electrode results in fairly diffuse RF energy flow, and, accordingly, minimal to nonexistent heating and cell damage adjacent the return electrode.

The turning on and turning off of ablation energy is monitored by the system through the storage of time on and time off information at step 418. The system collects this data as well as skin temperature data to determine what distribution of RF energy between a plurality of return electrodes will minimize the likelihood of patient discomfort and/or injury, as will be discussed in detail below.

The amount of power applied to each electrode may be varied, as may be the number of electrodes being excited with RF energy. Generally, enough energy must be passed through the return electrodes as is needed to ablate the particular tissue mass to be destroyed. Initially, the system may allocate the current to be passed through the return electrodes equally between all the return electrodes. Likewise, the system may automatically apply current to return electrodes. These initial operating parameters are subject to amendment as operating data is gathered and the operating parameters are adjusted to maximize the likelihood of patient comfort, by avoiding overheating.

Alternatively, choices may be based on operation type, surgeon identity (to accommodate for expected surgeon specific customary operating procedures), or other surgeon set preferences.

It is useful to monitor the impedance of the ablation system, and in particular the quality of the connection of the return electrode to the skin. This may be done in a number of ways. For example, upon the application of RF energy to an ablation device at step 416, the system may proceed to measure the current through one of the return electrodes at step 420. At the same time, the system captures voltage information from the radio frequency source and uses this information to calculate the impedance of the path between the ablation stylet and the return electrode. Generally, low impedance in the path between the ablation stylet and the return electrode signifies that a good connection has been made at the return electrode. If a bad connection is indicated, and an alarm sounded, the surgeon or an assistant then has the opportunity to reset or replace the electrode. Poor connection may be a primary cause of overheating. The high ohmic resistance of a poor connection tends to increase the amount of power sent to and dissipated by the electrode/skin interface.

The quality of an electrical connection at a return electrode is a function of numerous factors. For example, the skin may be dry, or moist. Likewise, oily skin or contaminant materials may tend to prevent adhesion Muscle movement and other mechanical factors may also affect the quality of the connection initially, and over time. Mechanical delamination of an otherwise good electrical connection may also result in a poor electrical connection.

Even for good connections, path impedance can vary depending upon numerous factors. For example, some areas of the body have relatively large fat deposits under the skin. Other areas may have mostly venous or muscle tissue. Still other areas will have bony structures, cartilage or the like. In addition, all of these factors will vary from individual to individual. Moreover, dependent upon the electrode placement, the path length will also change. All these factors will affect the ability of a return electrode to operate effectively and pass a desired magnitude of current.

At step 422, the resistance which results from all of these factors is measured and a determination made as toward whether the impedance is within an acceptable range. If it is determined that the impedance is outside the acceptable range, at step 424 an alarm is sounded. In addition, the particular electrode or electrodes with temperatures outside the range may be displayed on the display of the computer used to control the system. If desired, the physician may override the alarm and choose to accept the poor connection at step 426. Alternatively, all electrode temperatures may be displayed.

In either case, the system proceeds to step 428 where it is determined whether or not all electrodes have been tested. If they have not, the system increments to the next electrode at step 430 and measures the current through that electrode at step 431, after which the system returns to step 422 to repeat the sequence until all electrodes have been measured.

At the point where it is determined at step 428 that all electrodes have been measured, the system operates to apply RF ablation energy to the ablation electrodes. After a period of time, for example something in the range of 10 seconds to a few minutes, for example three minutes, the system proceeds again to step 432, where the temperature of the first return electrode is measured. As there has been a previous measurement, the determination is made at step 434 as toward whether there has been a temperature change. If there has been no temperature change, the system proceeds to step 436 where it is determined whether the temperatures of all electrodes have been measured. If the temperature of all electrodes have not been measured, the system proceeds to step 438, where it increments to the next electrode and returns to step 432 to repeat the temperature measurement. This cycle is performed until all electrodes have had their temperature measured, as is determined at step 436.

Once all electrodes have again had their temperature measured, an initial set of data is established, and the system proceeds, after a period of applying ablation current, at step 436 to step 432 to repeat the sequence of impedance and temperature measurement.

Each time that a temperature is measured at step 432, the system sends such measurement information to the computer at step 440. Likewise, each time the system determines that there has been a rise in temperature at step 434, the system proceeds to step 442, where the inventive system adjusting algorithm is implemented to control optionally the amount of RF ablation energy sent to each electrode and optionally which electrodes are receiving RF ablation energy.

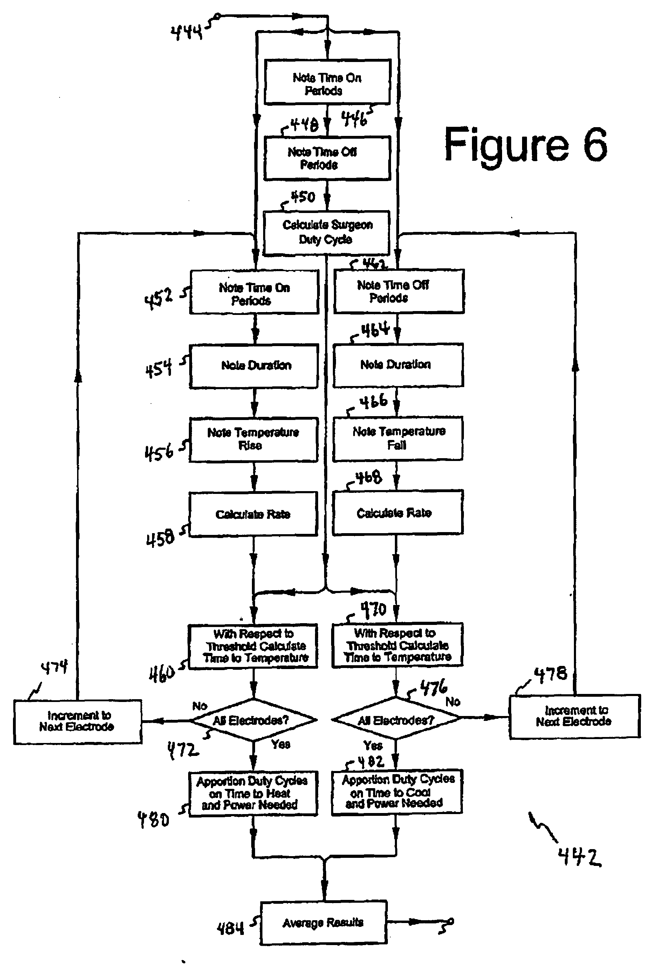

As illustrated in Figure 6, the power adjustment algorithm which begins at node 444 is implemented at step 434 upon the determination that there has been a change in electrode temperature. Alternatively, application of the power adjustment algorithm may be reserved for those occasions where there has been an increase (or, alternatively, a problematic increase) in electrode temperature.

The times at which the system turns on various return electrodes are noted at step 446. The times at which the system turns off are noted at step 448. This enables the calculation of an overall on time, during performance of a procedure which is specific to the particular operation. Potentially, from a database standpoint, this is storable as characteristic of the operating style of a particular surgeon. This calculation is done at step 450.

It is also an object of the present disclosure to measure the effect on electrode temperature of the presence or absence of RF ablation energy. Such effect may be difficult to predict, as it relates 1) to the quality of connection, 2) the nature of underlying tissue, 3) the cooling effect of the vasculature, 4) the insulative nature of tissue between the return electrode and ablation point, and 5) the quality of (i) the thermal coupling to, (ii) the thermal conductivity of, and (iii) the heat capacity of the tissue coupled to the return electrode.

The actual effect of the application of RF energy and its removal may be used to generate information for the allocation of ablation energy between return electrodes.

More particularly, by associating time periods during which electrodes are on at step 452, with the duration of those time periods at step 454, and with the magnitude of a temperature rise at step 456, the system may calculate the rate of temperature rise during the application of RF ablation energy at step 458.

Using this information, the system, at step 460, can calculate the time it will take for the temperature to rise to an unacceptable threshold level. This may be done for all electrodes. On this basis, the amount of excitation energy applied to each electrode may be apportioned in such a manner so as to make it more likely that the electrodes will all rise in temperature at the same rate. This criteria may be used as a sole criteria for controlling the amount of energy applied to the electrodes.

Such amount of energy may be controlled, for example, by varying the duty cycle of energy applied to the electrodes. By duty cycle is meant the sequential turning on and off of RF ablation power. It is also possible to vary an absolute amount of energy applied to the electrodes by varying the amplitude of the RF signal applied to the return electrode.

However, amplitude is maintained constant, but during the period during which the surgeon is causing ablation energy to enter a tissue mass to be destroyed, the system is rapidly turning on and off, for example at the rate of 10 Hz, with on-time constituting between, for example, 10% and 90% of the cycle, with 10% corresponding to a 10% application of the full power of the RF ablation source and 90% on-time corresponding to application of RF energy equal to 90% of the total power of the RF ablation source.

Alternatively or in addition, the assessment steps reflected by steps 462-470, by associating time periods during which electrodes are instructed to be off on account of the release of the RF actuating switch by the physician on the ablation device, at step 462, with the duration of those time periods at step 464, and with the magnitude of a temperature drop during such periods at step 466, the system may calculate the rate of temperature rise during the application of RF ablation energy at step 468.

Using this information, the system, at step 470, can calculate the time it will take for the temperature to fall to an acceptable threshold level. This may be done for all overheated electrodes. On this basis, the amount of excitation energy applied to each electrode may be apportioned in such a manner so as to make it more likely that the electrodes will all cool at the same rate, thus tending to ensure that they will not become overheated beyond the acceptable threshold.

As alluded to above, the allocations which the system begins to calculate at steps 462-470 may be based on an assumption that the electrodes are activated only 50% of the time by the surgeon. This initial assumption may be varied as information is gathered at steps 446-450 and combined with the rates calculated at steps 458 and 468 to calculate time to the threshold temperature.

As described generally above, the assessment of positive changes in electrode temperature is repeated by determining whether all electrodes have been assessed at step 472, incrementing to the next electrode at step 474 and repeating the process by returning to step 452.

Also as described generally above, the assessment of negative changes in electrode temperature is repeated by determining whether all electrodes have been assessed at step 476, incrementing to the next electrode at step 478 and repeating the process starting at step 462.

Apportionment of duty cycles is performed with respect to time to heat at step 480. In addition or in the alternative, an apportionment may be performed at step 482 with respect to time to cool. Results may be combined and averaged at step 484.

Referring back to Figure 5, after the power adjustment algorithm has been run at step 442, the system proceeds to step 486 where the new operating parameters are applied. The system then determines whether, at step 436, all electrodes have been measured and proceeds with the above described process until such measurements have been completed, after which the system proceeds to repeat the above process.

In connection with the same, it is noted that each time a temperature is sent to the computer at step 440, the system proceeds at step 488 to determine whether a particular electrode has exceeded the highest acceptable electrode temperature. If such is not the case, the system proceeds as discussed above.

However, if the threshold temperature has been exceeded, as determined at step 488, the system proceeds to disable the electrode at step 490. At the same time an alarm is sounded and the display on the computer indicates which electrode has been disabled at step 492. At step 494, the surgeon may override the automatic shutoff of the offending electrode. This is recognized by the system at step 496, after which the system proceeds to step 442 to restore the electrode to the system. If no override is received, at step 494 the system proceeds to adjust power allocations in accordance with the disabling of the subject electrode. During such a period ablation energy to other electrodes may be increased if their temperature permits this without danger or discomfort to the patient.

The system also displays at step 497 the temperature of the electrodes as measured at step 432. It is also contemplated that in response to such display, the surgeon may elect to change threshold temperature and this information is input into the system at step 498. Likewise, the computer with which the system is used may display the temperature setting for the highest acceptable electrode temperature at step 499.

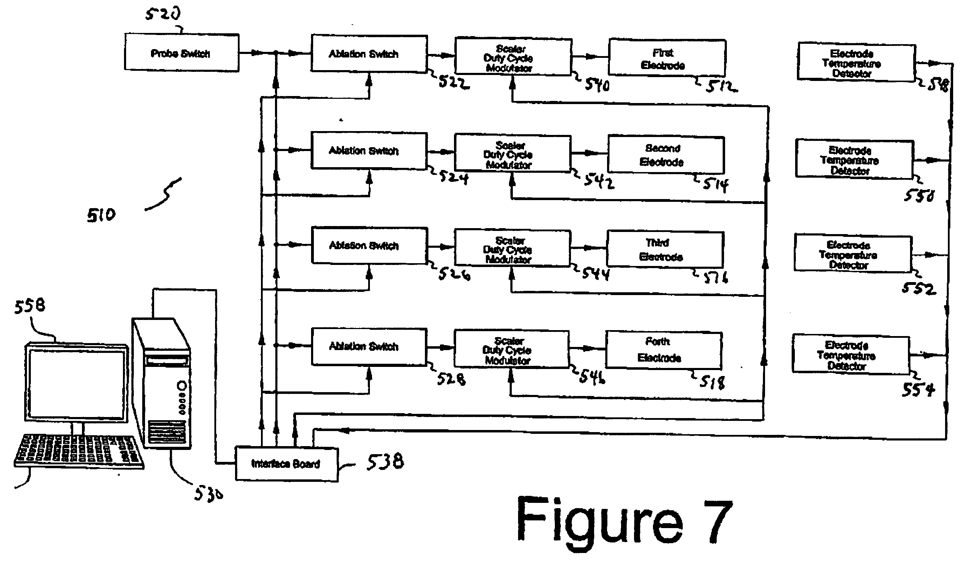

Referring to Figure 7, an apparatus according to the present invention is illustrated. System 510 comprises a plurality of electrodes 512-518. Upon the actuation of, for example, a push button 520 on the ablation apparatus, energy is coupled to a plurality of ablation switches 522-528. Switches 522-528 act as gates to couple the application of power in response to controls from computer 530 which provides such controls through an interface board 538.

Power is coupled from the ablation switches to respective duty cycle modulators 540-546, which output an intermittent RF signal whose duty cycle is controlled by computer 530 coupled to modulators 540-546 by interface board 538. This allows computer 530 to control the power sent to electrodes 512-518.

Such power is controlled in response to electrode temperature. The temperature of electrodes 512-518 is measured by temperature detectors 548-554, whose outputs are provided by interface board 538 to computer 530. Such temperature may be displayed on computer screen 556, along with other information, as described above in connection with the description of the example of Figures 5-6.

In addition, any alarms which need to be provided by the system, including audible alarms, may be provided by computer 530. At the same time visual alarms may be provided on screen 558, together with the other information described above in connection with the example of Figures 5-6.

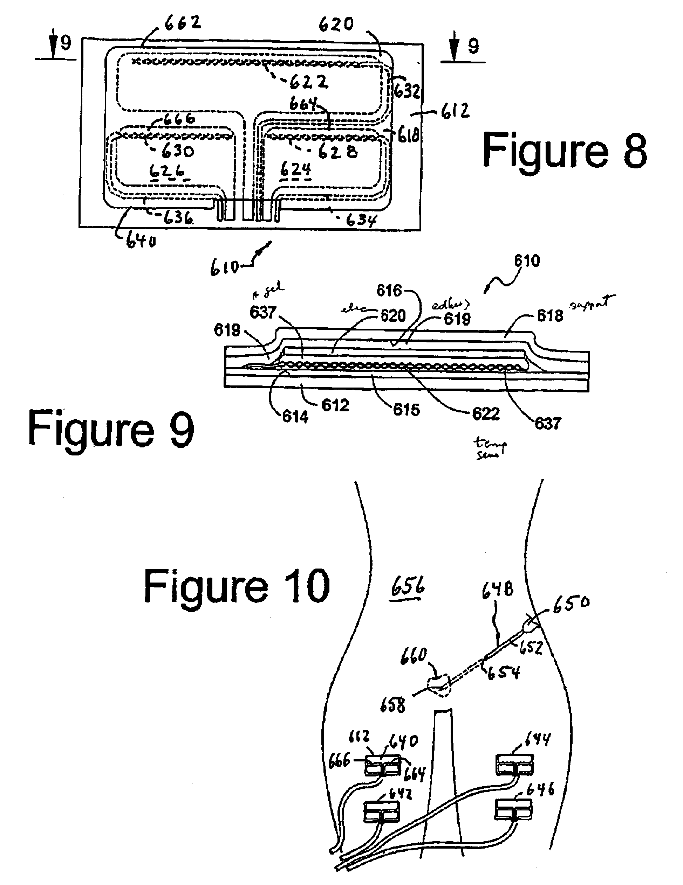

Referring to Figures 8-9, an electrode assembly 610 according to the present invention is illustrated. More particularly, it is contemplated that electrode pads may include more than one electrode and that the application of power to return electrodes may be varied as described above, with each electrode on a pad given its own separate temperature measurement transducer. Electrode assembly 610 is provided to the user with an adhesive protective member 612 whose surface 614 is coated with a release agent such as wax 615. The underside 616 of electrode support member 618, made for example of plastic film, foam or paper, is coated with adhesive material 619 which is protected by protective member 612.

A foil electrode 620 is secured to support member 618. A temperature sensing transducer 622 is, in turn, disposed over and secured to electrode 620 by adhesive 619. Foil and adhesive are of conventional thickness being about the thickness of common paper, but are exaggerated in size for purposes of illustration in the figures. In similar fashion, electrodes 624 and 626 are secured to and coupled to temperature sensing transducers 628 and 630. Transducers 622,628 and 630 are coupled respectively to lead wires 632,634 and 636, and are adhered in position by a layer of hydrogel 637, a conductive material used in skin contacting electrodes, and which overlies the entire surface of the electrodes that contact the skin during use.

Placement of four electrode assemblies 640, 642, 644 and 646 is illustrated in a typical configuration in Figure 10. An ablation device 648 comprising a handle 650 and trocar 652 is inserted through a hole 654 in the skin 656 of a patient with its point 658 in a tissue mass 660 which is to be ablated. Typically, stylets are caused to exit from trocar point 658. Radiofrequency ablation energy is then applied to the electrode assemblies and the stylets, causing a current path between the stylets adjacent point 658 and the electrode assemblies.

In accordance with the present invention it is contemplated that temperature sensing transducers, such as transducers 622, 628 and 630 are positioned at the edge of the electrodes closest to the point of application of RF energy by the stylets. Accordingly, transducers are positioned adjacent edges 662, 664 and 666 of assembly 640, as can be seen most clearly in Figure 8. Transducers are shown as twisted pair members, but an untwisted transducer comprising copper and constantan, side by side in an untwisted configuration may be used. The transducer may be on either side of the electrode. Accordingly, a transducer may alternatively be glued to the side of the foil electrode opposite the layer of hydrogel to be contacted with the skin.

The illustrated placement at the leading edge of the current path is made because the edge of each of the electrodes closest to the point of application of ablation energy adjacent point 658 tend to be the edge which conducts most of the RF ablation energy and thus tend to be the edge that heats up. Thus, temperature measurement is made of the hottest part of the electrode and the possibility of discomfort or injury minimized.

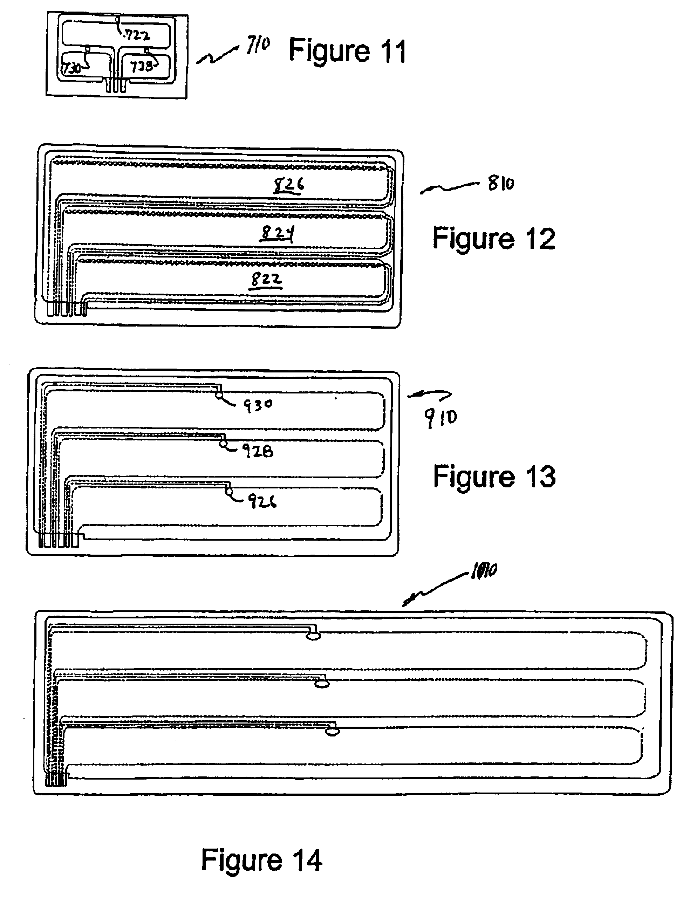

Referring to Figure 11, an alternative example of an electrode 710, however not forming part of the present invention, is illustrated. Electrode 710 is similar to electrode 610 illustrated in Figure 8, except that the temperature transducers 722, 728 and 730 are small circular members which are preferably, placed at the center of the leading edge of their respective electrodes.

Referring to Figure 12, a multi-electrode pad 810 including elongated electrodes 822, 824 and 826 is illustrated. The elongated electrodes are of particular value, insofar as they provide a very long leading edge. Each electrode 822, 824 and 826 may be, for example, in the range of approximately 4 cm in width and 20 cm long. Otherwise, this structure is similar to that of Figure 8.

It is contemplated that small electrodes, such as electrodes 926, 928, and 930 may be incorporated into an electrode 910.

The length of an electrode 1010 may be varied as required by the power output of the system. For example, a very wide electrode 1010, such as that illustrated in Figure 14 may be used in applications where it is desirable to use a relatively high powered ablation device without too much off time.

It is contemplated that the distribution of power between return electrodes may be varied in accordance with return electrode temperature, electrode impedance, and the combination of the return electrode temperature and impedance. Power may be intermittently applied in accordance with a fixed function independent of return electrode temperature and impedance. Alternatively, intermittent application of power may be added to any of the above strategies to provide a failsafe in the event of selective electrode delamination, temperature measurement failure and/or impedance measurement failure.

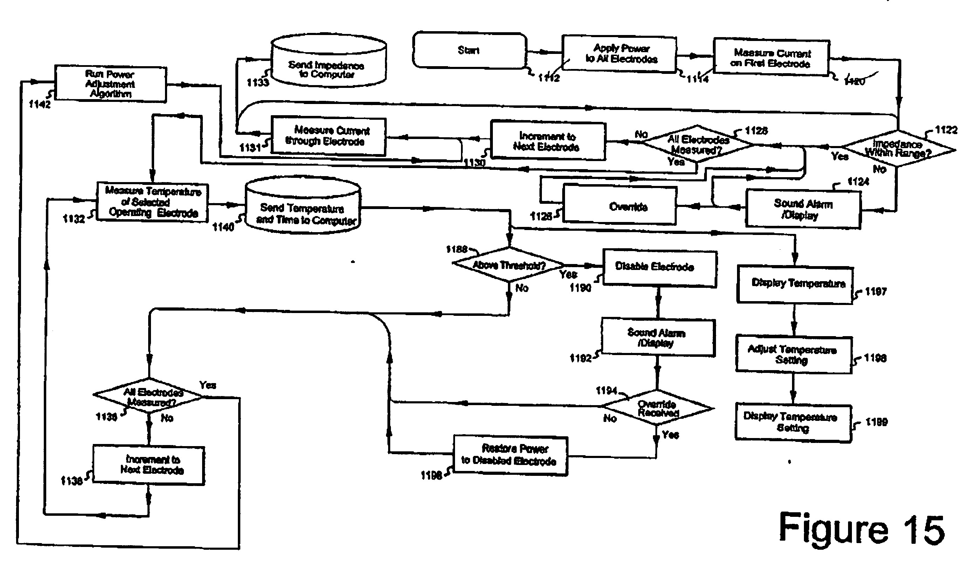

Referring to Figure 15, a system in which the distribution of power between return electrodes may be varied in accordance with the combination of the return electrode temperature and impedance, is illustrated. The operation of the system illustrated in Figure 15 is similar to that of the system illustrated in Figure 5, except that in addition to monitoring impedance for the purpose of providing an alarm to the user with respect to, for example, delamination, this information is, additionally, used to generate control information for controlling the distribution of power between the return electrodes.

In accordance with the system illustrated in Figure 15, after the start of the system at step 1112 followed by the application of impedance testing power, at operation 1114, between, for example, separate electrode members of a single pair, the system proceeds to measure current through the first electrode at operation 1120.

The system then proceeds through a loop of impedance measurement at operation 1122, a determination whether all electrodes have been measured at operation 1128, incrementation at operation 1130 and repeated measurement of current at operation 1131 until all electrodes have been measured. This is determined at operation 1128, and upon the determination that all return electrodes have had their impedance measured, the system proceeds to a second loop involving temperature measurement at operation 1132, storage of temperature and time within a computer at operation 1140, comparison to a threshold at operation 1188, a determination whether all electrodes have been measured at operation 1136 and incrementating to the next electrode at operation 1138.

Temperature display, temperature setting, and a display of temperature setting is provided at operations 1197,1198 and 1199.

In the event that it is determined at operation 1188 that a temperature is above the threshold, the system proceeds to disable the electrode at operation 1190 and sound an alarm at operation 1192. In the manner of the example of Figure 5, an override may be detected at operation 1194, resulting in a reversal of the command to disable electrode power. In either alternative, the system then proceeds to continue the return electrode temperature measurement cycle.

When the temperature measurement cycle has been completed, as determined at operation 1136, the system proceeds to operation 1142 where a power adjustment algorithm is used to determine the distribution of power between the various return electrodes. The system then operates with the determined power allocation for a period of time, after which the impedance measurement and electrode temperature measurements may be repeated, and power reallocated, if necessary.

A number of different methodologies may be employed to determine the allocation of power between return electrodes. Generally, it is contemplated, where possible (for example possible without causing a patient undue pain or injury) at least one electrode will always be receiving power, and thus all the ablation stylets are substantially continuously receiving ablation energy. Typically, it is contemplated that return electrode power will be alternated between various return electrodes, thus giving them the possibility of being driven with an ablation current which, for example, during alternating periods of time, is low enough, optionally zero, to give the body a chance to cool through such mechanisms as blood flow and conduction.

It is contemplated that the algorithm for adjusting power in response to temperature measurements, for example temperature measurements over time, may be any of the methodologies and algorithms described above.

In the case of impedance, power may be allocated inversely in accordance with impedance, for example, with a proportional distribution of power impedance, with the lowest impedance electrodes receiving the most return electrode power and the highest impedance electrodes receiving the least return electrode power. This power adjustment algorithm based on impedance may be used, advantageously, on systems which do not incorporate temperature sensitive transducers for monitoring temperature. Such a system would have the advantage of using inexpensive electrode pads without temperature transducers.

Alternatively, the allocation of return electrode power may be responsive to both temperature and impedance. Optionally, the allocation of power may be responsive to both temperature and impedance and subjected to predetermined off periods for electrodes regardless of temperature or impedance as a failsafe measure.

If it is desired to control the allocation of power between return electrodes in response to both electrode temperature and the impedance of the connection between the electrode and the skin, the above algorithms for determination of allocation in response to temperature may be separately calculated to obtain a first scaling factor for each electrode. The allocation in response to skin electrode impedance noted above may also be separately calculated to obtain an impedance responsive scaling factor for each electrode. The individual temperature and impedance scaling factors for each electrode may then be multiplied by each other to determine the actual scaling factor to be employed.

Optionally, the allocation of power between electrodes may be between pads, or, alternatively, between individual electrodes on a single pad or, as a yet further alternative, between individual electrodes regardless of pad location.

In addition to this, cut offs may be employed with electrodes having an impedance above a certain value being shut down and resulting in an alarm.

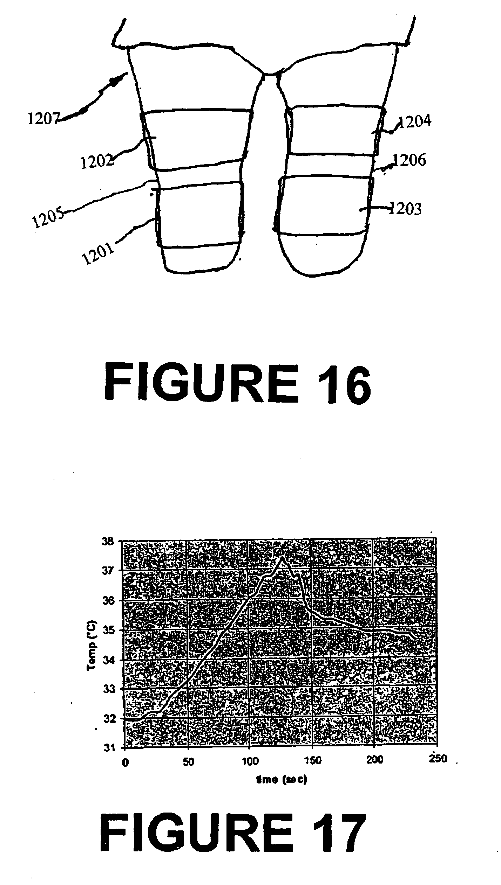

Electrodes, for example any one of electrodes 1201-1204 illustrated in Figure 16, having a size of, for example, 12.5 cm in width and 25 cm in length, may be applied to the thighs 1205 or 1206 of a human subject 1207. More particularly, applied power to the electrodes may be multiplexed with one electrode receiving power while the others not receiving power and alternative the application of power to the return electrodes. Preferably power may be continuously applied to the ablation electrode, with power being applied to each of the electrodes individually for only a portion of that period of time during which power is applied to the ablation electrode.

Upon the application of a current to the electrodes, heating, over a period of approximately 130 seconds was noted as illustrated in Figure 17. Upon the removal of the current, cooling occurred as illustrated in Figure 17, with initial cooling being more rapid than heating or long-term cooling.

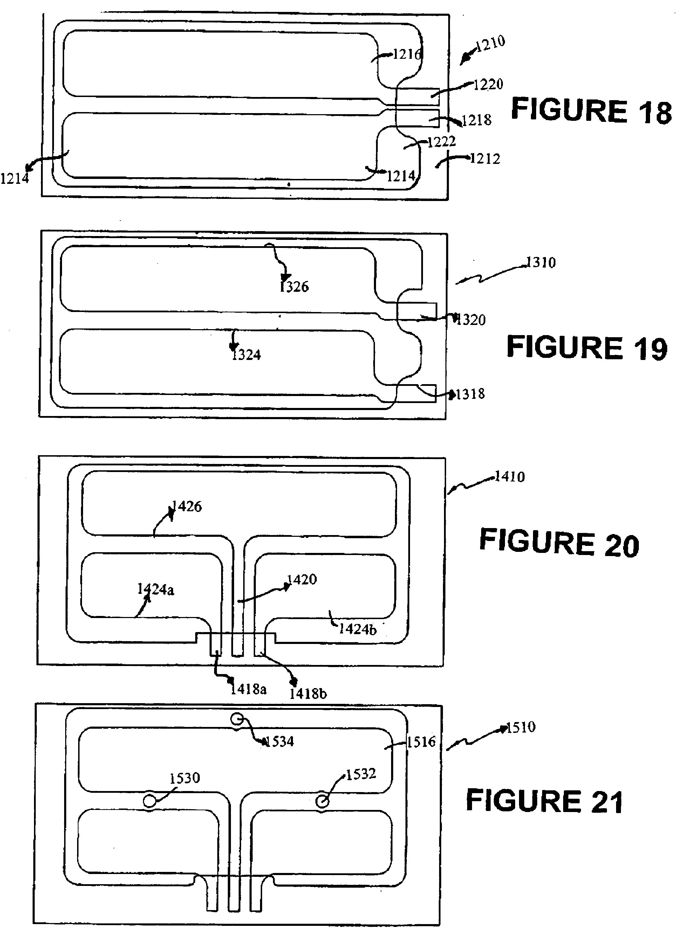

Preliminary tests involved application of Aaron Medical (Bovie) ESRE-1 return electrodes, similar to those illustrated in Figure 18, to the anterior of the two thighs for test purposes only. That is to say, current was applied between electrodes for test purposes, rather than between an electrode or electrodes and an ablation needle. Tests were conducted on three different subjects (1 female, 2 male). RF current at approximately 460 kHz, produced by a Rita Medical ablation system was used.

Referring to Figure 18, a return electrode is illustrated. Electrode 1210 comprises a base 1212 with a pair of electrodes 1214 and 1216. A lead 1218 is associated with and integral with electrode 1214, optionally being stamped from a single sheet of conductive material, such as copper. A lead 1220 is associated with and integral with electrode 1216, optionally also being stamped from a single sheet of conductive material, such as copper.

Electrodes 1214 and 1216 are held in position by an adhesive layer mounted on a support member 1222. Support member 1222 may be made of fabric, plastic or any other suitable material. As illustrated in Figure 18, base 1212 is coated with a release material which adheres to the adhesive layer supported on support member 1222. Before use, base 1212 is removed, exposing the adhesive layer. Electrodes 1214 and 1216 are adhered to the same layer of adhesive which adheres to the skin of the thigh, for example, of a subject, and brings the electrodes 1214 and 1216 into contact with, for example, the thigh of the subject

During a test, using an electrode similar to that illustrated in Figure 18, resistance was first measured between the two halves of the split electrode, namely electrodes 1214 and 1216. The resistance from one electrode half 1214 to the other electrode half 1216 was approximately 52 ohms for the female subject and 20 to 24 ohms for the male subjects. When measuring the resistance from an electrode on one leg to an electrode on the other leg, the total resistance was measured at approximately 79 ohms for the female subject and 67 to 68 ohms for the male subjects.

It appears that it may be difficult to determine how well the return electrode contacts are made by measuring resistance from one pad to the other (one leg to the other). However, in the relatively small sample tested, the difference from leg to leg was only about 11 to 12 ohms (about 15% of the total leg to leg resistance). Yet the resistances between the pad halves differed by more than a factor of two. Accordingly, it is believed that measuring the resistance between the halves of a split electrode is a better indicator of contact integrity.

The resistances involved at the pad sites and between the pads and the procedure location (the point at which the monopolar ablation electrode is applied) thus appear to be a significant portion of the total resistance. Thus, only a fraction of the total power applied is available to do the intended work at the procedure location.

In a series of tests conducted with the Rita Medical RF source, a Flir Systems A40M infrared camera was used to monitor temperature.

Resistance from pad-half to pad-half was measured at 19 to 24 Ohms (about the same as measured previously on this subject) Resistance measured from left leg to right leg was measured at approximately 60 Ohms (slightly lower than measured previously)

Referring to Figure 16, two ESRE-1 pads were placed on each leg, forming an array of four electrodes (electrode 1 on one thigh nearest the foot, electrode 1202 nearest the torso on the same thigh, and electrode 1203 nearest the foot and electrode 1204 nearest the torso on the other thigh). The resistance between immediately adjacent electrodes (electrode 1201 and electrode 1202 in one case, and electrode 1203 and electrode 1204 in another) was 23 to 24 Ohms. The resistance between alternate electrodes (electrode 1201 and electrode 1204 or electrode 1202 and electrode 1203) was 36 to 40 Ohms. With the four-electrode array just described, a current of 2 Amps between alternate electrodes (electrode 1202 and electrode 1204) could not be tolerated for more than a few seconds. Discomfort was felt in the vicinity of the cable connections.

At 1.4 Amps, the subject could tolerate the application of current for a bit longer, but not for more than about 30 seconds. When 1.4 Amps was applied for 15 seconds, and then off for 15 seconds, this procedure could be tolerated for 4 minutes or more. This apparently gives the tissue under the electrode being used time to cool down. While the subject felt discomfort in the area under the electrode pad connections (such as leads 1218 and 1220), a thermal camera did not indicate that there was more heating adjacent the electrode pad connections. However, it may be that some subcutaneous effects were being felt. It is noted that this was with ESRE-1 electrode pads oriented laterally, i.e., as illustrated in Figure 16.

As illustrated in Figure 17, when an RF current of approximately 0.7 amps was applied between immediately adjacent electrodes (e.g., electrode 1201 and electrode 1202, the rate of rise of temperature over time was observed to be less than the rate of fall, at least over some time interval, indicating that time multiplexing the electrodes has a beneficial effect heating.

In the test illustrated in Figure 17, an RF current (0.7 Amps) was turned on for about 10-20 seconds, and was allowed to run for 2 minutes. The current was then turned off and the skin temperature allowed to return toward equilibrium. The initial skin temperature was about 32 °C. The temperature rose linearly at about 0.05 °C/second. After the RF current was turned off, the temperature fell at about 0.087 °C/second for about 15 seconds. The temperature did not return to the original (32 °C) value until long after the current flow stopped.

It was observed that when current is applied from one pad to the other the hottest parts are along the line between the pads. There is considerably less heating in portions of the electrode progressively further from the edge of the pad that is in closest proximity to the other part of the circuit.

Generally, it was observed that the portion of the edge (the "leading edge") of an electrode closest to an electrode (which simulates the ablation needle) conducts substantially most of the current, thus resulting in that edge developing considerable heat in the adjacent portion of the skin.

The problem with the configuration illustrated in Figure 18 is that the electrical connections are at one side. If the upper part is conducting, the "leading edge" is away from the connection point. However, if current is flowing to the lower part, the leading edge is along the line that includes the connection part or lead. This appears to contribute to the discomfort felt by the subject.

An alternative electrode pad 1310 with a different configuration is illustrated in Figure 19. Here, leading edges 1324 and 1326 are opposite leads 1318 and 1320, promoting patient comfort.

In yet another alternative electrode 1410, illustrated in Figure 20, lower left and right lower electrode sections 1424a and 1424b, as well as electrode 1426 are connected together (by a conductor in a conventional clamp connector) to effectively form a single long electrode. This is done because the connector grasps all three leads 1418a, 1418b and 1420.

Referring to Figure 21, yet another alternative electrode 1510 is illustrated. In this example, three spots are provided for thermocouples 1530,1532, and 1534. Thermocouple 1534 is provided at the center of the upper part, and thermocouples 1530 and 1532 are provided along the leading edge of the lower part of the electrode 1516. This configuration has the advantage of monitoring the temperature along each electrode edge, and also monitoring at three positions laterally. If the electrode pad is being "iced" with an ice pack, but the ice pack is not being applied uniformly, there is thus a better chance of detecting the error and alerting the physician.

## Claims

1. An ablation system for ablating a biological mass (660) in a patient, comprising:
(a) a source of electrical ablation energy having first, second and third power outputs;
(b) a first conductor coupled to said first power output on said source of electrical energy;
(c) a second conductor coupled to said second power output on said source of electrical energy, said source of electrical energy creating a first output ablation voltage between said first and second power outputs, said first output ablation voltage varying between a first higher average value during a first period of time and a first lower average value for a second period of time, said first lower average value being greater than or equal to zero;
(d) a third conductor coupled to a third power output on said source of electrical energy, said source of electrical energy creating a second output ablation voltage between said first and third power outputs, said second output ablation voltage varying between a second higher average value during a third period of time and a lower average value for a fourth period of time, said lower average value being greater than or equal to zero;
(e) an ablation probe (648), said ablation probe being coupled to said first conductor, and said ablation probe comprising a trocar (652) and a plurality of stylets extending from a pointed tip (658) of the trocar;
(f) a first and a second return electrode (620, 624, 626), each return electrode having a straight leading edge (662, 664, 666), wherein the return electrodes are configured to be positioned on a patient's skin, such that each leading edge is that edge of a return electrode that is closest to the point of application of ablation energy;
(g) a curved edge being contiguous to the first and second ends of each leading edge of said return electrodes; and
(h) temperature sensors (622, 628, 630), each secured to and coupled to a respective return electrode to provide temperature measurement of the return electrodes, wherein power of the electrical ablation energy source is controlled in response to the electrode temperature to alleviate overheating of the skin of the patient;
**characterized in that**
the temperature sensors (622, 628, 630) are positioned adjacent and along said leading edges (662, 664, 666).

2. An ablation system as in claim 1, wherein said first period of time overlaps a substantial portion of said fourth period of time, and said second period of time overlaps a substantial portion of said third period of time.

3. An ablation system as in claims 1 or 2; wherein said first return electrode and said second return electrode are disposed on a common substrate, said common substrate being configured to be
adhered to the skin of said patient.

4. An ablation system as in claims 1 or 2, wherein said first and second return electrodes are configured to be positoned on one leg of said patient, and further comprising third and fourth return electrodes configured and driven with power as recited in claim 1, said third and fourth return electrodes being configured to be positioned on the other leg of said patient.

## Patentansprüche

1. Ablationssystem zur Ablation von biologischer Masse (660) in einem Patienten, das umfasst:
(a) eine Quelle elektrischer Ablationsenergie mit einem ersten, zweiten und dritten Leistungsausgang;
(b) einen ersten Leiter, der mit dem ersten Leistungsausgang an dieser Quelle elektrischer Energie verbunden ist;
(c) einen zweiten Leiter, der mit dem zweiten Leistungsausgang an der Quelle elektrischer Energie verbunden ist, wobei die Quelle elektrischer Energie eine erste Ablations-Ausgangsspannung zwischen dem ersten und zweiten Leistungsausgang erzeugt, diese erste Ablations-Ausgangsspannung zwischen einem ersten höheren Durchschnittswert während einer ersten Zeitspanne und einem ersten niedrigeren Durchschnittswert für eine zweite Zeitspanne variiert, wobei der erste niedrigere Durchschnittswert größer oder gleich Null ist;
(d) einen dritten Leiter, der mit einem dritten Leistungsausgang an der Quelle elektrischer Energie verbunden ist, wobei die Quelle elektrischer Energie eine zweite Ablations-Ausgangsspannung zwischen dem ersten und dritten Leistungsausgang erzeugt, diese zweite Ablations-Ausgangsspannung zwischen einem zweiten höheren Durchschnittswert während einer dritten Zeitspanne und einem niedrigeren Durchschnittswert für eine vierte Zeitspanne variiert, wobei der niedrigere Durchschnittswert größer oder gleich Null ist;
(e) eine Ablationssonde (648), wobei diese Ablationssonde mit dem ersten Leiter verbunden ist und diese Ablationssonde einen Trokar (652) und eine Vielzahl von Führungsstäben umfasst, die sich von einer kegelig zulaufenden Spitze (658) des Trokars erstrecken;
(f) ein erste und eine zweite Neutralelektrode (620, 624, 626), wobei die Neutralelektroden jeweils eine gerade Vorderkante (662, 664, 666) aufweisen und dazu konfiguriert sind, derart auf der Haut eines Patienten angeordnet zu werden, dass die Vorderkanten jeweils jene Kanten der Neutralelektroden sind, die sich am nächsten zu dem Anwendungspunkt der Ablationsenergie befinden;
(g) eine gewölbte Kante, die sich jeweils an das erste und zweite Ende der Vorderkanten dieser Neutralelektroden anschließt; und
(h) Temperatursensoren (622, 628, 630), die jeweils an den Neutralelektroden befestigt und damit verbunden sind, um eine Temperaturmessung der Neutralelektroden zu bieten, wobei die Leistung der Quelle elektrischer Ablationsenergie in Reaktion auf die Elektrodentemperatur gesteuert wird, um ein Überhitzen des Haut des Patienten zu lindern;
**dadurch gekennzeichnet, dass**
die Temperatursensoren (622, 628, 630) angrenzend an und entlang den Vorderkanten (662, 664, 666) angeordnet sind.

2. Ablationssystem nach Anspruch 1, wobei die erste Zeitspanne einen wesentlichen Teil der vierten Zeitspanne überlappt und die zweite Zeitspanne einen wesentlichen Teil der dritten Zeitspanne überlappt.

3. Ablationssystem nach Anspruch 1 oder 2, wobei die erste Neutralelektrode und die zweite Neutralelektrode auf einem gemeinsamen Substrat angeordnet sind und das gemeinsame Substrat dazu konfiguriert ist, an die Haut des Patienten geklebt zu werden.

4. Ablationssystem nach Anspruch 1 oder 2, wobei die erste und zweite Neutralelektrode dazu konfiguriert sind, auf einem Bein des Patienten angeordnet zu werden, und das des Weiteren eine dritte und vierte Neutralelektrode umfasst, die nach Anspruch 1 konfiguriert sind und angesteuert werden, wobei die dritte und vierte Neutralelektrode dazu konfiguriert sind, auf dem anderen Bein des Patienten angeordnet zu werden.

## Revendications

1. Système d'ablation destiné à l'ablation d'une masse biologique (660) chez un patient, comprenant :
(a) une source d'énergie électrique d'ablation ayant des première, deuxième et troisième sorties de puissance ;
(b) un premier conducteur accouplé à ladite première sortie de puissance sur ladite source d'énergie électrique ;
(c) un deuxième conducteur accouplé à ladite deuxième sortie de puissance sur ladite source d'énergie électrique, ladite source d'énergie électrique créant une première tension d'ablation de sortie entre lesdites première et deuxième sorties de puissance, ladite première tension d'ablation de sortie variant entre une première valeur moyenne supérieure durant une première période et une première valeur moyenne inférieure pour une deuxième période, ladite première valeur moyenne inférieure étant supérieure ou égale à zéro ;
(d) un troisième conducteur accouplé à une troisième sortie de puissance sur ladite source d'énergie électrique, ladite source d'énergie électrique créant une deuxième tension d'ablation de sortie entre lesdites première et troisième sorties de puissance, ladite deuxième tension d'ablation de sortie variant entre une deuxième valeur moyenne supérieure durant une troisième période et une valeur moyenne inférieure pour une quatrième période, ladite valeur moyenne inférieure étant supérieure ou égale à zéro ;
(e) une sonde d'ablation (648), ladite sonde d'ablation étant accouplée au dit premier conducteur, et ladite sonde d'ablation comprenant un trocart (652) et une pluralité de stylets s'étendant à partir d'une extrémité pointue (658) du trocart ;
(f) des première et deuxième électrodes de retour (620, 624, 626), chaque électrode de retour ayant un bord avant droit (662, 664, 666), dans lequel les électrodes de retour sont configurées pour être positionnées sur la peau d'un patient, de telle sorte que chaque bord avant soit le bord d'une électrode de retour qui est le plus proche du point d'application de l'énergie d' ablation ;
(g) un bord incurvé étant contigu avec les première et deuxième extrémités de chaque bord avant desdites électrodes de retour ; et
(h) des capteurs de température (622, 628, 630), chacun fixé et accouplé à une électrode de retour respective pour fournir une mesure de température des électrodes de retour, dans lequel la puissance de la source d'énergie électrique d'ablation est régulée en réponse à la température d'électrode pour atténuer une surchauffe de la peau du patient ;
**caractérisé en ce que**
les capteurs de température (622, 628, 630) sont positionnés à côté et le long desdits bords avant (662, 664, 666).

2. Système d'ablation selon la revendication 1, dans lequel ladite première période chevauche une partie sensible de ladite quatrième période, et ladite deuxième période chevauche une partie sensible de ladite troisième période.

3. Système d'ablation selon les revendications 1 ou 2, dans lequel ladite première électrode de retour et ladite deuxième électrode de retour sont disposées sur un substrat commun, ledit substrat commun étant configuré pour être collé à la peau dudit patient.

4. Système d'ablation selon les revendications 1 ou 2, dans lequel lesdites première et deuxième électrodes de retour sont configurées pour être positionnées sur une jambe dudit patient, et comprenant en outre des troisième et quatrième électrodes de retour configurées et entraînées avec la puissance selon la revendication 1, lesdites troisième et quatrième électrodes de retour étant configurées pour être positionnées sur l'autre jambe dudit patient.
